Europäisches Patentamt

European Patent Office

Office européen des brevets

(11) Veröffentlichungsnummer: **0 262 420**

**A2**

(12) **EUROPÄISCHE PATENTANMELDUNG**

(21) Anmeldenummer: 87112634.8

(22) Anmeldetag: 29.08.87

(51) Int. Cl.4: **C07C 139/00** , C07C 143/10 , C07C 69/30 , C07C 69/24 , C11D 1/28 , C11D 10/02

(30) Priorität: 11.09.86 DE 3630931
05.08.87 DE 3725935

(43) Veröffentlichungstag der Anmeldung:
06.04.88 Patentblatt 88/14

(84) Benannte Vertragsstaaten:
AT BE CH DE ES FR GB GR IT LI LU NL SE

(71) Anmelder: Akzo N.V.
Postbus 186 Velperweg 76
NL-6800 LS Arnhem(NL)

(72) Erfinder: Bunzel, Wolfgang, Dr.
Trockener Weiher 85
D-5190 Stolberg/Rheinland(DE)
Erfinder: Jansen, Franz
Lucherberger Strasse 30
D-5176 Inden-Pier(DE)

(74) Vertreter: Fett, Günter
Akzo GmbH Kasinostrasse 19-23
D-5600 Wuppertal 1(DE)

(54) Oberflächenaktive Kondensationsprodukte.

(57) Oberflächenaktive Kondensationsprodukte werden hergestellt durch Verestern von Carbonsäure der Formel RCOOH mit einem hydroxyalkansulfonsaurem Salz der Formel $HO-(CH_2)_n-SO_3X$, wobei n Werte von 2 bis 4 annehmen kann und X ein Alkalimetall oder $NH_4$ bedeutet, in Gegenwart von Konsistenzreglern, wobei als Konsistenzregler Ester von Carbonsäuren mit 3-36 Kohlenstoffatomen mit Alkoholen mit 3 bis 30 Kohlenstoffatomen verwendet werden. Geeignet sind Ester wie Isopropylstearat, Cetylstearat sowie entsprechende Palmitate, auch können Fettsäureester des Glycerins und anderer mehrwertiger Alkohole eingesetzt werden. Auch Ester von Dicarbonsäuren sind geeignet. Die Konsistenzregler regeln günstig den Verfahrensablauf und erlauben u.a. ein besseres Abdestillieren von überschüssiger Carbonsäure. Die Konsistenzregler können im Endprodukt verbeiben, da sie wertvolle Inhaltsstoffe sind.

EP 0 262 420 A2

## Oberflächenaktive Kondensationsprodukte

Die Erfindung betrifft ein Verfahren zur Herstellung von oberflächenaktiven Kondensationsprodukten durch Verestern von Carbonsäuren mit Salzen von Hydroxyalkansulfonsäuren.

Die Herstellung derartiger Kondensationsprodukte ist bereits seit langem bekannt. So wird in dem Buch von August Chwala, Textilhilfsmittel, Ihre Chemie, Kolloid-Chemie und Anwendung Verlag J. Springer, Wien, 1939 auf den Seiten 173 bis 174 ein solches Verfahren beschrieben. Dabei wird Ölsäure oder Ölsäurechlorid mit hydroxyäthansulfonsaurem Natrium verestert. Der dabei entstehende Ester der Ölsäure und der Hydroxyäthansulfonsäure (Isäthionsäure), auch Igepon genannt, ist oberflächenaktiv und hat sich z.B. als Waschmittel für Proteinfasern und Kunstfasern aus regenerierter Cellulose bewährt.

Die Bedeutung derartiger Kondensationsprodukte hat im Laufe der Zeit zugenommen, und so gibt es zahlreiche Hinweise auf derartige Herstellungsverfahren, sei es in der Patentliteratur oder in Fachzeitschriften.

In der DE-OS 3 442 579 wird ein Verfahren zur Herstellung von oberflächenaktiven Kondensationsprodukten durch Verestern von Carbonsäuren mit Salzen von Hydroxyalkansulfonsäuren beschrieben, bei dem die Veresterung in Gegenwart eines Konsistenzreglers durchgeführt wird. Als Konsistenzregler sind nach dieser Offenlegungsschrift Ester synthetischer oder natürlicher Fettsäuren besonders geeignet, wobei der Methylester derartiger Säuren bevorzugt wird. Außer Methylester werden in dieser Offenlegungsschrift noch Äthylester erwähnt. Unter Konsistenzreglern sind nach Lehre dieser Offenlegungsschrift solche Verbindungen zu verstehen, die die Viskosität des Reaktionsgemisches herabsetzen. Bevorzugt werden solche Konsistenzregler eingesetzt, die nach der Beendigung der Umsetzung weitgehend aus dem Endprodukt entfernt werden können oder die im Endprodukt keine schädlichen oder unerwünschten Nebenwirkungen entfalten.

Da bei der Herstellung der Kondensationsprodukte überschüssige Fettsäure abdestilliert wird, verarmt gegen Ende der Umsetzung das Reaktionsgemisch bzw. das erhaltene Kondensationsprodukt auch zunehmend an Konsistenzregler, so daß die Viskosität und der Erstarrungspunkt des flüssigen Reaktionsgemisches ansteigt und die Gefahr besteht, daß das Gemisch fest wird, wenn die erforderliche Temperatur nicht genau eingehalten wird und es unbeabsichtigt zu einer Abkühlung des Gemisches kommt.

Das Abdestillieren des Konsistenzreglers erfordert zusätzlich Energie; außerdem ist es aufwendig, das abdestillierte Gemisch von Konsistenzregler und Fettsäuren für den Wiedereinsatz aufzuarbeiten.

Es besteht deshalb noch ein Bedürfnis nach einem verbesserten Verfahren, das die o.g. Nachteile nicht aufweist und zu Produkten mit wertvollen Eigenschaften führt.

Aufgabe der Erfindung ist es deshalb, ein Verfahren der eingangs beschriebenen Art zur Verfügung zu stellen, das sich einfach durchführen läßt, bei dem insbesondere die Viskositätsverhältnisse während, gegen Ende und nach Abschluß der Veresterung günstig sind, das es erlaubt, auf einfache Weise die überschüssige Fettsäure nach der Veresterung aus dem oberflächenaktiven Produkt zu entfernen und das über eine verbesserte Prozeßsicherheit verfügt.

Aufgabe der Erfindung ist es ferner, ein Verfahren zur Verfügung zu stellen, das mit Konsistenzreglern arbeitet, die im Endprodukt verbleiben können und die die Eigenschaften der Endprodukt in vorteilhafter Weise beeinflussen.

Aufgabe der Erfindung ist es ferner ein Verfahren zur Verfügung zu stellen, das zu Produkten führt, die insbesondere für den Einsatz bei kosmetischen Formulierungen geeignet sind.

Diese Aufgabe wird gelöst durch ein Verfahren zur Herstellung von oberflächenaktiven Kondensationsprodukten durch Verestern von Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der Formel $HO-(CH_2)_n-SO_3X$, wobei n Werte von 2 bis 4 annehmen kann und X ein Alkalimetall oder $NH_4$ bedeutet, in Gegenwart eines Konsistenzreglers, das dadurch gekennzeichnet ist, daß man als Konsistenzregler Ester von Carbonsäuren mit 3 bis 36 Kohlenstoffatomen und Alkoholen mit 3 bis 30 Kohlenstoffatomen verwendet. Sehr geeignet sind Ester mit insgesamt 17-44 Kohlenstoffatomen.

Vorzugsweise werden Ester von gesättigten und/oder ungesättigten Monocarbonsäuren mit 8 bis 18 Kohlenstoffatomen verwendet. Sehr geeignet sind auch Ester von mehrwertigen Alkoholen, insbesondere Triglyceride.

Auch können Ester von Dicarbonsäure eingesetzt werden.

Bevorzugt werden 1 bis 5 Gw.-% des Konsistenzreglers eingesetzt, wobei die Gewichtsmenge bezogen wird auf das bei der Kondensation erhaltene Endprodukt.

Es können auch in vorteilhafter Weise Gemische der Ester verwendet werden. Man kann auch zwei oder mehrere verschiedene Konsistenzregler verwenden und diese zu verschiedenen Zeitpunkten des Verfahrens zusetzen.

Das Herstellungsverfahren der oberfächenaktiven Kondensationsprodukte durch Verestern von Carbonsäure mit Salzen von Hydroxyalkansulfonsäuren gemäß der Erfindung kann grundsätzlich auf die Art und Weise durchgeführt werden, wie es in der DE-OS 3 442 579 beschrieben wird. Im Gegensatz zu der Verwendung von Methyl-oder Äthylestern als Konsistenzreglern wird jedoch erfindungsgemäß im allgemeinen eine geringere Menge des Konsistenzreglers eingesetzt, wobei für viele Einsatzzwecke Mengen von 1 bis 5 Gw.-%, bezogen auf das als Endprodukt entstehende Kondensationsprodukt, ausreichend sind.

Je nach gewünschten Eigenschaften des Endproduktes kann diese Menge jedoch erhöht werden, so sind Mengen bis zu 10 bis 15 %, ja sogar bis zu 50 und bis zu 100 % und mehr möglich.

Wenn auch die Menge des verwendeten Konsistenzreglers in weiten Grenzen variiert werden kann, ist es in vielen Fällen vorteilhaft, wenn man die Menge des eingesetzten Konsistenzreglers davon abhängig macht, welche Menge man im fertigen Endprodukt wünscht bzw. akzeptieren kann.

Erfindungsgemäß können die Konsistenzregler bereits von Anfang an den Ausgangsstoffen zugemischt werden, sie können aber auch im Verlauf der Umsetzung oder gegen Ende der Verfahrens hinzugefügt werden. Auch eine portionsweise Zugabe während der Veresterung ist möglich. Man kann auch zwei oder mehrere verschiedene Konsistenzregler verwenden und diese getrennt oder in Mischung zugeben.

Der Zusatz der Konsistenzregler ist auch möglich vor der Destillationsphase, d.h. wenn die Veresterungsreaktion schon weitgehend abgeschlossen ist und man den Überschuß an eingesetzter Carbonsäure und evtl. noch vorhandenes Wasser abdestillieren möchte.

Als geeignete Konsistenzregler im Rahmem der Erfindung seien beispielsweise angeführt Isopropylstearat, Isobutylstearat, 2-Äthylhexylstearat, Cetylstearat, Isocetylstearat sowie die entsprechenden Palmitate, Myristate oder Laurate sowie die Ester von verzweigten und unverzweigten primären, sekundären oder tertiären, einwertigen oder mehrwertigen Alkoholen mit 3 bis 30 Kohlenstoffatomen von diesen oder anderen Fettsäuren; so sind Fettsäureester des Glycerins, Trimethylolpropans oder des Pentaerytrits geeignet, ferner Walrat und Fettsäuretriglyceride. Ebenfalls geeignet sind Dicarbonsäureester wie z.B. Di-2-äthylhexyladipat.

Weitere in vorteilhafter Weise verwendbare Konsistenzregler sind beispielsweise Jojobaöl, Bienenwachs, Lanolinester, Spermaceti oder Kakaobutter.

Die vorstehend erwähnten Ester sowie ähnliche, namentlich nicht genannte Verbindungen können, soweit sie als Naturstoffe existieren, sowohl als Naturstoffe als auch in ihrer synthetischen Form eingesetzt werden.

Durch den Zusatz der erfindungsgemäß eingesetzten Konsistenzregler wird während der Veresterung nicht nur die Konsistenz des Reaktionsansatzes auf günstigen Werten gehalten, sondern es ist auch nicht notwendig, die Konsistenzregler nach Abschluß der Kondensation aus dem Reaktionsgemisch zu entfernen, da sie vor allem in Körperpflegemitteln wertvolle Inhaltsstoffe sind, die im Endprodukt verbleiben können.

Es war besonders überraschend, daß durch den erfindungsgemäßen Einsatz des Konsistenzreglers die Sicherheit des Herstellungsverfahrens erheblich verbessert wird. So wird bei Einsatz von Methylester als Konsistenzregler gegen Ende der Reaktion und nach Abdestillieren der Hauptmenge der überschüssigen Fettsäure und des Konsistenzreglers der Erstarrungspunkt der Schmelze heraufgesetzt, wobei ein Ansteigen der Viskosität festzustellen ist. Wird gegen Ende der Reaktion die Temperatur nicht besonders genau kontrolliert und geregelt und kommt es zu unvorhergesehenen Abkühlungen, so kann die Schmelze erstarren, so daß es zum Rührerstillstand kommt.

Bei Einsatz von Estern von Carbonsäure mit Alkoholen mit 3 bis 30 Kohlenstoffatomen hingegen wird der Temperaturbereich, innerhalb dessen die Reaktionsschmelze flüssig bleibt, erheblich vergrößert, so daß auch Temperaturschwankungen von 5 bis 10°C oder mehr nicht zum Erstarren der Schmelze führen.

Es ist ferner möglich, den Gehalt an freien Fettsäuren durch Abdestillieren viel stärker zu reduzieren, als das bei Verwendung von Methyl-oder Äthylestern als Konsistenzregler der Fall ist. Dadurch werden niedrigere Säurezahlen und hohe Gehalte an WAS erreicht. Erfindungsgemäß ist es möglich, den Reaktionsansatz praktisch voll auszudestillieren.

Es war ferner überraschend, daß es gemäß der Erfindung möglich ist, mit wesentlich geringeren Mengen an Konsistenzregler auszukommen.

Da man bei Einsatz von Methyl-und Äthylestern verhältnismäßig große Mengen einsetzen muß, um möglichst viel freie Fettsäuren abzudestillieren, war es nicht zu erwarten, daß man gleiche oder sogar wesentlich höhere Mengen an Fettsäuren abdestillieren kann bei Einsatz von wesentlich niedrigeren Mengen des erfindungsgemäß verwendeten Konsistenzreglers. Auf diese

Weise ist es möglich, die Raumzeitausbeute eines Reaktors zu verbessern. Man kann deshalb entweder den Umsatz z.B. bei chargenweiser Reaktionsführung um 15 bis 20 % erhöhen bzw. man kann bei gleichen Umsatzmengen mit 15 bis 20 % kleiner ausgelegten Anlagen arbeiten. Dadurch wird nicht nur Raum gespart sondern auch Energie.

Für manche Einsatzzwecke ist ein sprühgetrocknetes Produkt erwünscht, da dieses Vorteile gegenüber nicht sprühgetrockneten Materialien aufweisen kann und im allgemeinen auch besser rieselfähig ist. Es hat sich gezeigt, daß das gemäß der Erfindung hergestellte Produkt besonders gut sprühgetrocknet werden kann, da es weitgehend frei ist von Verbindungen, die bei einem nachgeschalteten Sprühtrocknungsprozeß in die Abluft gelangen und zu Geruchsbelästigungen führen.

Ein weiterer Vorteil des erfindungsgemäßen Verfahrens besteht darin, daß die verwendeten Konsistenzregler wertvolle Inhaltsstoffe darstellen, die das Produkt insbesondere für kosmetische Anwendungen geeignet machen.

Besonders vorteilhaft ist es, daß man mit dem erfindungsgemäßen Verfahren sehr genau einen bestimmten WAS-Gehalt und einen bestimmten Inhaltsstoffgehalt reproduzierbar einstellen kann. So kann man z.B. durch Zusatz entsprechender Mengen von Isopropylstearat als Konsistenzregler zu den Ausgangsprodukten den Gehalt an Isopropylstearat im Endprodukt genau festlegen und ein Produkt erhalten, das über eine sehr hohe WAS verfügt. Es ist jedoch nicht möglich, ein entsprechendes Produkt durch nachträgliches Konfektionieren zu erhalten, z.B. indem man gemäß dem Verfahren entsprechend der DE-OS 3,442,479 ein oberflächenaktives Kondensationsprodukt herstellt und nachträglich das fertige Kondensationsprodukt mit einer entsprechenden Menge an Isopropylstearat vermischt. Zwar kann man durch nachträgliches Vermischen Produkte mit dem gleichen Gehalt an Isopropylstearat herstellen, wie es gemäß der Erfindung möglich ist. Produkte, welche gemäß der Erfindung hergestellt werden, weisen jedoch gegenüber Produkten, die durch nachträgliches Vermischen von Kondensationsprodukt und Isopropylstearat gewonnen sind, eine beachtlich höhere WAS auf.

Von weiterem Vorteil ist, daß man gemäß der Erfindung den Gehalt der Inhaltsstoffe genau einstellen kann. Oberflächenaktive Kondensationsprodukte, welche gemäß der Erfindung hergestellt worden sind, lassen sich auch noch nachträglich mit Inhaltsstoffen verschneiden und ergeben Gemische, die sich durch hohe WAS auszeichnen.

Von Vorteil ist ferner, daß die bei der Durchführung des Verfahrens abdestillierten Fettsäuren nicht von mitabdestillierten Konsistenzreglern abgetrennt werden müssen, sondern ohne größere Probleme wiederverwendet werden können.

Die mit dem erfindungsgemäßen Verfahren erreichbaren sehr niedrigen Säurezahlen (10 bis 20), die sehr guten Farb-und Geruchsqualitäten erlauben es, das erhaltene Produkt besonders vorteilhaft in kosmetischen Formulierungen, z.B. Seifen, einzusetzen. Die mitverwendeten Konsistenzregler wirken sich mit ihren hautpflegenden Eigenschaften besonders vorteilhaft aus.

Das erfindungsgemäß Verfahren arbeitet somit besonders sicher, was die Prozeßführung betrifft, es ist wirtschaftlich und umweltschonend. Darüber hinaus führt es zu Produkten mit verbesserten Eigenschaften, die sich insbesondere bei Körperpflegeprodukten sehr vorteilhaft bemerkbar machen.

Bisweilen kann es zweckmäßig sein, um die Eigenfarbe des erhaltenen Produkts zu verbessern, ein Bleichmittel wie Sulfit, insbesondere Natriumsulfit, zuzusetzen.

Es ist ferner möglich, auch nach dem Destillieren noch Ester zuzugeben um den Gehalt an Ester im oberflächenaktiven Produkt zu erhöhen. Dabei wird eine gute Verteilung des Zusatzes erreicht und eine gute Homogenität des Produkts erzielt.

Auch läßt sich die Staubbildung, die bei verschiedenen Kondensationsprodukten gemäß dem Stand der Technik zu beobachten ist, erheblich reduzieren.

Die Erfindung wird durch folgende Beispiele näher erläutert:

Beispiel 1

In einem 1 l-Dreihaltskolben werden 222 Teile Kokosfettsäure, 125,8 Teile Natriumhydroxyethansulfonat und 5,5 Teile Phosphorsäure unter $N_2$-Schutzgas gerührt und innerhalb von 2 Stunden auf 235°C aufgeheizt. Man hält für 4 Std. bei dieser Temperatur und destilliert das entstehende Reaktionswasser zusammen mit geringen Fettsäureanteilen ab. Nach 4 Std. werden 11,7 Teile Isopropylstearat hinzugefügt und nach Homogenisieren vorsichtig Vakuum angelegt, um das Produkt durch Abdestillieren der überschüssigen Fettsäure und geringen Anteilen an Ester und Reaktionswasser aufzukonzentrieren. Bei einem Enddruck von 40 mbar wird das Vakuum mit $N_2$ aufgehoben. Das Reaktionsprodukt bleibt beim Abkühlen bis ca. 220°C rührfähig.

Kennzahlen : WAS    81,9%
SZ    26,1
Klettfarbe    14

Beispiel 2

1,25 Mol Fettsäure (Kokosfettsäure) werden mit 1 Mol Na-Hydroxyäthansulfonat umgesetzt. Zu dem Reaktionsgemisch werden von Beginn an 5 Gew.-% eines Triglycerids aus Glycerin und Fettsäuren mit 8 bis 9 Kohlenstoffatomen, berechnet auf die entstehende Menge Kondensationsprodukt gegeben. Nach anschließender Vakuumdestillation wird ein Produkt erhalten, das folgende Kennzahlen hat:
SZ = 16
WAS = 83,9%

Beispiel 3

Auf gleiche Weise wird ein Kondensationsprodukt unter der Verwendung von 5 Gew.-% Isocetylstearat als Konsistenzregler hergestellt. Das Endprodukt weist folgende Daten auf:
SZ = 19
WAS = 82,8%

**Ansprüche**

1. Verfahren zur Herstellung von oberflächenaktiven Kondensationsprodukten durch Verestern von Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der Formel HC-$(CH_2)_n$-$SO_3X$, wobei n Werte von 2 bis 4 annehmen kann und X ein Alkalimetall oder $NH_4$ bedeutet, in Gegenwart eines Konsistenzreglers, dadurch gekennzeichnet, daß man als Konsistenzregler Ester von Carbonsäuren mit 3 bis 36 Kohlenstoffatomen und Alkoholen mit 3 bis 30 Kohenstoffatomen verwendet.

2. Verfahren nach Anspruch 1, dadurch gekennzeichnet, daß man Ester mit insgesamt 17 bis 44 Kohlenstoffatomen verwendet.

3. Verfahren nach Anspruch 1 oder 2, dadurch gekennzeichnet, daß man Ester von gesättigten und/oder ungesättigten Monocarbonsäuren mit 8 bis 18 Kohlenstoffatomen verwendet.

4. Verfahren nach einem der Ansprüche 1 bis 3, dadurch gekennzeichnet, daß man Ester mehrwertiger Alkohole verwendet.

5. Verfahren nach Anspruch 4, dadurch gekennzeichnet, daß man Triglyceride verwendet.

6. Verfahren nach Anspruch 1 oder 2, dadurch gekennzeichnet, daß man Ester von Dicarbonsäuren verwendet.

7. Verfahren nach einem der Ansprüche 1 bis 6, dadurch gekennzeichnet, daß man 1 bis 5 Gew.-% Konsistenzregler verwendet.

8. Verfahren nach einem der Ansprüche 1 bis 7, dadurch gekennzeichnet, daß man Gemische von Konsistenzreglern verwendet.

9. Verfahren nach einem der Ansprüche 1 bis 8, dadurch gekennzeichnet, daß man zwei oder mehrere verschiedene Konsistenzregler verwendet und diese zu verschiedenen Zeitpunkten des Verfahrens zusetzt.

10. Verwendung der nach einem Verfahren gemäß einem der Ansprüche 1 bis 9 hergestellten oberflächenaktiven Kondensationsprodukt bei der Herstellung von kosmetischen Formulierungen.